# EUROPEAN PATENT APPLICATION

(11) **EP 4 817 601 A1**
(43) Date of publication of application: **07.10.2026**
(21) Application number: 24898124.3
(22) Date of filing: 27.11.2024
(51) Int. Cl.: A61K 9/51, A61K 47/64, A61K 9/00, A61K 31/436, A61P 35/00

(54) **NANOPARTICLES COMPRISING SIROLIMUS AND ALBUMIN, PHARMACEUTICAL COMPOSITION FOR SUBCUTANEOUS ADMINISTRATION, COMPRISING SAME, AND PREPARATION METHOD THEREFOR**

(30) Priority: 29.11.2023 KR 20230170005
(71) Applicant: SNBioscience Inc., Seongnam-si, Gyeonggi-do, 13449 (KR)
(72) Inventor: PARK, Young-hwan, Seoul 06374 (KR); JUNG, Jaehun, Seoul 04587 (KR); KO, Bosung, Seongnam-si Gyeonggi-do 13383 (KR)
(74) Representative: J A Kemp LLP
(86) International application number: PCT/KR2024/019007
(87) International publication number: WO 2025/116530

(57) **Abstract**

The present disclosure relates to nanoparticles comprising sirolimus and albumin, a pharmaceutical composition for subcutaneous administration comprising the same, and a method for preparing the same. When the preparation method of the present disclosure is used, nanoparticles comprising sirolimus and albumin having excellent particle size distribution maintenance stability can be prepared, and the prepared nanoparticles can be prepared into a pharmaceutical composition suitable for subcutaneous injection, thereby improving an increase in an administration dose and the convenience of medication administration.

## Description

### Technical Field

The present disclosure is the result of an application filed with the support of the '2023 Global Startup Commercialization Support Program' of Gyeonggi Province and the Gyeonggi Business & Science Accelerator.

This application claims priority to Korean Patent Application No. 10-2023-0170005, filed with the Korean Intellectual Property Office on November 29, 2023, and the disclosure of the patent application is incorporated by reference in this application.

The present disclosure relates to nanoparticles comprising sirolimus and albumin, a pharmaceutical composition for subcutaneous administration comprising the same, and a method for preparing the same.

### Background Art

Sirolimus is a mammalian target protein of rapamycin called mTOR (mammalian target of rapamycin) is a conserved serine/threonine kinase that plays a central role in intracellular signaling. Activation of the mTOR pathway is associated with cell proliferation and survival, and inhibition of mTOR signaling leads to inflammation and cell death. Because dysregulation of the mTOR signaling pathway is related to human diseases including cancer and autoimmune disorders, it is used in a wide range of applications to treat solid tumors, hematological malignancies, organ transplantation, restenosis, and rheumatoid arthritis by using mTOR inhibitors such as sirolimus. Sirolimus, which has a synonym of Rapamycin, is being marketed as a product called Rapamune tablet, and is being used for preventing organ rejection in kidney transplant patients and treating patients with lymphangioleiomyomatosis. Because it is a poorly soluble drug, its bioavailability (Biovailability, BA) is very low at 14%, and it has been developed into tablets and liquid formulations for intravenous injection. However, in the case of the intravenous injection formulation, since the time required for administration takes as little as 30 minutes, and as much as 60 to 24 hours, there is a problem of low patient compliance, and it has a disadvantage of a high intensity of side effects due to a high initial blood concentration.

### Technical Problem

The inventors of the present disclosure have made intensive research efforts to develop a nanoparticle formulation of sirolimus that solves the above-mentioned problems. As a result, they completed the present disclosure by identifying that when nanoparticles are prepared by binding albumin to sirolimus and adding hyaluronidase, the yield is high, the size of the nanoparticles does not abruptly change upon reconstitution in an aqueous solvent, and it is also suitable for subcutaneous injection.

Accordingly, an aspect of the present disclosure is to provide a method for preparing nanoparticles comprising sirolimus and albumin.

Another aspect of the present disclosure is to provide nanoparticles comprising sirolimus, albumin, and hyaluronidase.

Still another aspect of the present disclosure is to provide a pharmaceutical composition for treating cancer, comprising the above-described nanoparticles.

### Solution to Problem

The present disclosure provides the inventions of the following 1 to 22.
1. A method for preparing nanoparticles comprising sirolimus and albumin, comprising the following steps:
   (a) preparing a first solution in which sirolimus is dissolved at 5 to 75 w/v%;
   (b) preparing a second solution in which albumin is dissolved at 5 to 30 w/v%;
   (c) mixing and stirring the first solution and the second solution having a volume ratio of 1:5 to 1:70; and
   (d) diluting with an aqueous solvent in an amount of 2 to 10 times based on the volume of the solution having passed through step (c).
2. The method for preparing nanoparticles according to 1, further comprising (e) removing an organic solvent by drying the diluted solution under reduced pressure.
3. The method for preparing nanoparticles according to 2, wherein the drying under reduced pressure is performed under a temperature condition in a range of 10 to 70°C, 20 to 70°C, 30 to 70°C, 40 to 70°C, 10 to 60°C, 10 to 50°C, 10 to 40°C, 20 to 60°C, 30 to 50°C, 35 to 45°C, 38 to 42°C, or 39 to 41°C.
4. The method for preparing nanoparticles according to 2 or 3, wherein the drying under reduced pressure is performed under a pressure condition in a range of 10 to 110 hPa, 20 to 110 hPa, 30 to 110 hPa, 40 to 110 hPa, 50 to 110 hPa, 10 to 100 hPa, 10 to 90 hPa, 10 to 80 hPa, 10 to 70 hPa, 20 to 100 hPa, 30 to 90 hPa, 40 to 80 hPa, 50 to 70 hPa, or 55 to 65 hPa.
5. The method for preparing nanoparticles according to any one of 1 to 4, further comprising (f) obtaining nanoparticles by filtering and drying the solution having passed through step (e).
6. The method for preparing nanoparticles according to any one of 1 to 5, wherein the drying is lyophilization, and a lyoprotectant is added in the lyophilization process.
7. The method for preparing nanoparticles according to 6, wherein hyaluronidase is added in the lyophilization process.
8. The method for preparing nanoparticles according to any one of 1 to 7, wherein the albumin is selected from the group consisting of human serum albumin (HSA), bovine serum albumin (BSA), ovalbumin (OVA), recombinant human serum albumin (rHSA), and combinations thereof.
9. The method for preparing nanoparticles according to any one of 1 to 8, wherein the step (c) of mixing and stirring the first solution and the second solution comprises a high-pressure homogenization process.
10. The method for preparing nanoparticles according to 9, wherein the high-pressure homogenization is performed under a pressure condition of 5,000 to 30,000 psi.
11. The method for preparing nanoparticles according to any one of 1 to 10, wherein the solvent of the first solution is selected from the group consisting of ethanol, methanol, isopropyl alcohol, butanol, dimethyl sulfoxide (DMSO), dimethylformamide (DMF), tetrahydrofuran (THF), acetonitrile, dichloromethane, ethyl acetate, hexane, diethyl ether, benzene, chloroform, acetone, and combinations thereof.
12. The method for preparing nanoparticles according to any one of 1 to 11, wherein the solvent of the second solution is an aqueous solvent selected from the group consisting of water, distilled water, sterile water, phosphate-buffered saline (PBS), methanol, purified water, ethanol, 1-propanol, 2-propanol, 1-pentanol, 2-butoxyethanol, ethylene glycol, acetone, 2-butanone, 4-methyl-2-propanone, chloroform, and combinations thereof.
13. The method for preparing nanoparticles according to any one of 1 to 12, wherein the step (d) is performed under a temperature condition of 1 to 40°C.
14. Nanoparticles comprising sirolimus, albumin, and hyaluronidase.
15. The nanoparticles according to 14, wherein the sirolimus and albumin are electrostatically bound to each other.
16. The nanoparticles according to 14 or 15, wherein a weight ratio of the sirolimus and albumin is 1:5 to 1:20.
17. The nanoparticles according to any one of 14 to 16, further comprising a lyoprotectant.
18. The nanoparticles according to any one of 14 to 17, wherein the nanoparticles are prepared by the preparation method of 1 to 13.
19. The nanoparticles according to any one of 14 to 18, wherein the albumin is selected from the group consisting of human serum albumin (HSA), bovine serum albumin (BSA), ovalbumin (OVA), recombinant human serum albumin (rHSA), and combinations thereof.
20. The pharmaceutical composition according to 20, wherein the pharmaceutical composition is a subcutaneous administration formulation, and is a pharmaceutical composition for treating cancer or for use as an immunosuppressant.
21. The pharmaceutical composition according to 20, wherein the pharmaceutical composition is a subcutaneous administration formulation, and is a pharmaceutical composition for treating cancer or for an immunosuppressant aspect.
22. A method for treating cancer, comprising administering the nanoparticles of any one of 14 to 19; or the pharmaceutical composition of 20 or 21 to a subject in need of treatment.

According to an aspect of the present disclosure, the present disclosure provides a method for preparing nanoparticles comprising sirolimus and albumin, comprising the following steps:
(a) preparing a first solution in which sirolimus is dissolved at 5 to 75 w/v%;
(b) preparing a second solution in which albumin is dissolved at 5 to 30 w/v%;
(c) mixing and stirring the first solution and the second solution having a volume ratio of 1: 5 to 1: 70; and
(d) diluting with an aqueous solvent in an amount of 2 to 10 times based on the volume of the solution having passed through step (c).

As used in this application, the term "about" used for numerical limitations, for example, temperature, time, pressure, amount, concentration, etc., including a range, may mean (+) or (-) 10%, 5%, 4%, 3%, 2%, 1%, or a range or a specific numerical value therebetween.

As used in this application, the term "nanoparticles" refers to particles having a particle size (dimension) of less than 1 µm. When the nanoparticles of the present disclosure are for intravenous injection, the nanoparticles preferably have a size of 500 nm or less. Specifically, the nanoparticles preferably have a size of 50-500 nm, 50-400 nm, 50-300 nm, 50-200 nm, 50-190 nm, 50-180 nm, 50-170 nm, 50-160 nm, 50-150 nm, 80-500 nm, 80-400 nm, 80-300 nm, 80-200 nm, 80-190 nm, 80-180 nm, 80-170 nm, 80-160 nm, 80-150 nm, 100-500 nm, 100-400 nm, 100-300 nm, 100-200 nm, 100-190 nm, 100-180 nm, 100-170 nm, 100-160 nm, 100-150 nm, 110-500 nm, 110-400 nm, 110-300 nm, 110-200 nm, 110-190 nm, 110-180 nm, 110-170 nm, 110-160 nm, or 110-150 nm.

In one embodiment of the present disclosure, the concentration of sirolimus in the first solution may be, for example, 5 to 75%(w/v), 5 to 70%, 5 to 65%, 5 to 60 %(w/v), 5 to 55 %(w/v), 5 to 50 %(w/v), 5 to 45 %(w/v), 5 to 40 %(w/v), 5 to 35 %(w/v), 5 to 30 %(w/v), 5 to 25 %(w/v), 5 to 20 %(w/v), 5 to 15 %(w/v), 5 to 10 %(w/v), 10 to 75 %(w/v), 15 to 75 %(w/v), 20 to 75 %(w/v), 25 to 75 %(w/v), 30 to 75 %(w/v), 35 to 75 %(w/v), 40 to 75 %(w/v), 45 to 75 %(w/v), 50 to 75 %(w/v), 55 to 75 %(w/v), 60 to 75%(w/v), 65 to 75%(w/v), 70 to 75%(w/v), 10 to 65 %(w/v), 15 to 60 %(w/v), 20 to 55 %(w/v), or 25 to 50 %(w/v), 30 to 45%(w/v), but is not limited thereto. Preferably, it may be 10 to 60%(w/v).

In one embodiment of the present disclosure, the solvent of the first solution is selected from the group consisting of ethanol, methanol, isopropyl alcohol, butanol, dimethyl sulfoxide (DMSO), dimethylformamide (DMF), tetrahydrofuran (THF), acetonitrile, dichloromethane, ethyl acetate, hexane, diethyl ether, benzene, chloroform, acetone, and combinations thereof, but is not limited thereto.

In one embodiment of the present disclosure, when the concentration of sirolimus in the first solution is less than the lower limit of the range of 5 to 75 %(w/v) defined in the present disclosure, even if the water dilution step is performed, a problem occurs in which the average particle size rapidly increases to 200 nm or more upon rehydration.

In addition, when the concentration of sirolimus in the first solution exceeds the upper limit of the range of 5 to 75 %(w/v) defined in the present disclosure, sirolimus precipitates during the nanoparticle preparation process, making the normal operation of the high-pressure homogenizer impossible, and the particle size of the nanoparticles also increases, thereby causing a problem in which nanoparticles of uniform particle size cannot be produced at a concentration exceeding 75 %(w/v).

In one embodiment of the present disclosure, the concentration of albumin in the second solution may be, for example, 5 to 30 %(w/v), 5 to 25 %(w/v), 5 to 20 %(w/v), 5 to 15 %(w/v), 5 to 10 %(w/v), 10 to 30 %(w/v), 15 to 30 %(w/v), 20 to 30 %(w/v), 25 to 30 %(w/v), 10 to 25 %(w/v), 15 to 20 %(w/v), but is not limited thereto.

In one embodiment of the present disclosure, the solvent of the second solution is an aqueous solvent selected from the group consisting of water, distilled water, sterile water, phosphate-buffered saline (PBS), methanol, purified water, ethanol, 1-propanol, 2-propanol, 1-pentanol, 2-butoxyethanol, ethylene glycol, acetone, 2-butanone, 4-methyl-2-propanone, chloroform, and combinations thereof, but is not limited thereto.

When the albumin concentration of the second solution is less than the lower limit of the range of 5 to 30 %(w/v) defined in the present disclosure, the volume of the dispersion in the finally prepared nanoparticles excessively increases, and accordingly, the filling amount increases to an extent that commercial production is impossible due to the low content. As a result, since it deviates from the typical filling volume range, an additional concentration process is required, thereby causing a problem of lowering economical efficiency and effectiveness. In addition, when the albumin concentration of the second solution exceeds the upper limit of the range of 5 to 30 %(w/v) defined in the present disclosure, even if the water dilution step is performed, a problem occurs in which the average particle size rapidly increases to 200 nm or more upon rehydration.

The volume ratio of the first solution in which the sirolimus is dissolved and the second solution in which the albumin is dissolved may be 1: 5 to 1: 70. For example, the volume ratio of the first solution and the second solution may be 1: 5 to 1: 70, 1: 5 to 1: 65, 1: 5 to 1: 60, 1: 5 to 1: 55, 1: 5 to 1: 50, 1: 5 to 1: 45, 1: 5 to 1: 40, 1: 5 to 1: 35, 1: 5 to 1: 30, 1: 5 to 1: 25, 1: 5 to 1: 20, 1: 5 to 1: 15, 1: 5 to 1: 10, 1: 10 to 1: 70, 1: 15 to 1: 70, 1: 20 to 1: 70, 1: 25 to 1: 70, 1: 30 to 1: 70, 1: 35 to 1: 70, 1: 40 to 1: 70, 1: 45 to 1: 70, 1: 50 to 1: 70, 1: 55 to 1: 70, 1: 60 to 1: 70, 1: 65 to 1: 70, 1: 10 to 1: 65, 1: 15 to 1: 60, 1: 20 to 1: 55, 1: 25 to 1: 50, or 1: 30 to 1: 45, but is not limited thereto.

In consideration of the reactivity and stability of the finally prepared nanoparticles, the volume ratio of the first solution and the second solution may be appropriately adopted such that the weight ratio of sirolimus:albumin becomes 1:5 to 1:20. For example, the weight ratio of the sirolimus:albumin may be 1:5 to 1:20, 1:5 to 1:18, 1:5 to 1:16, 1:5 to 1:14, 1:5 to 1:12, 1:5 to 1:10, 1:5 to 1:8, 1:7 to 1:20, 1:9 to 1:20, 1:11 to 1:20, 1:13 to 1:20, 1:15 to 1:20, 1:17 to 1:20, 1:6 to 1:17, 1:6.5 to 1:14, 1:7 to 1:11, 1:5 to 1:20, 1:5 to 1:20, 1:5 to 1:20, 1:5 to 1:20, 1:5 to 1:20, 1:5 to 1:20, 1:5 to 1:20, 1:5 to 1:20, 1:5 to 1:20, 1:5 to 1:20, but is not limited thereto.

In one embodiment of the present disclosure, the dilution in the step (d) may be diluted to an amount of 2 to 12 times, 2 to 10 times, 2 to 8 times, 2 to 6 times, 2 to 4 times, 4 to 12 times, 6 to 12 times, 8 to 12 times, 10 to 12 times, 4 to 10 times, or 6 to 8 times based on the volume of the suspension, but is not limited thereto. Preferably, it may be an amount of 2 to 10 times.

When the used multiple amount of the aqueous solvent used for dilution is less than the lower limit of the range of 2 to 10 times defined in the present disclosure, even if the water dilution step is performed, a problem occurs in which the particle size rapidly increases to 200 nm or more prior to drying under reduced pressure. The increase in the particle size may be due to agglomeration between nanoparticles. When the nanoparticles agglomerate, it may affect the efficacy and safety of the pharmaceutical composition comprising the nanoparticles. Accordingly, a process for removing the agglomerates is required, and since this immediately leads to a decrease in yield, economical efficiency and effectiveness are decreased.

In addition, when the used multiple amount of the aqueous solvent exceeds the upper limit of the range of 2 to 10 times defined in the present disclosure, not only does the unencapsulation rate of sirolimus increase, but more water than necessary is used for stabilizing the particle size of the nanoparticles, thereby causing a problem of being inefficient and uneconomical in terms of the scale-up process.

In one embodiment of the present disclosure, the step (d) may be performed under a temperature condition of 1 to 40°C. For example, it may be 1 to 40°C, 1 to 35°C, 1 to 30°C, 1 to 25°C, 1 to 20°C, 1 to 15°C, 1 to 10°C, 1 to 5°C, 5 to 40°C, 10 to 40°C, 15 to 40°C, 20 to 40°C, 25 to 40°C, 30 to 40°C, 35 to 40°C, 5 to 35°C, 10 to 30°C, or 15 to 25°C, but is not limited thereto.

In one embodiment of the present disclosure, the preparation method further comprises (e) removing an organic solvent by drying the diluted solution under reduced pressure.

When the drying according to one embodiment of the present disclosure is drying under reduced pressure, it may be performed by adopting a conventional method for drying under reduced pressure, such as using a rotary evaporator, and is not limited to specific conditions for drying under reduced pressure.

In a specific embodiment of the present disclosure, the drying under reduced pressure may be performed under a temperature condition in a range of 10 to 70°C, 20 to 70°C, 30 to 70°C, 40 to 70°C, 10 to 60°C, 10 to 50°C, 10 to 40°C, 20 to 60°C, 30 to 50°C, 35 to 45°C, 38 to 42°C, or 39 to 41°C, but is not limited thereto.

In a specific embodiment of the present disclosure, the drying under reduced pressure may be performed under a pressure condition in a range of 10 to 110 hPa, 20 to 110 hPa, 30 to 110 hPa, 40 to 110 hPa, 50 to 110 hPa, 10 to 100 hPa, 10 to 90 hPa, 10 to 80 hPa, 10 to 70 hPa, 20 to 100 hPa, 30 to 90 hPa, 40 to 80 hPa, 50 to 70 hPa, or 55 to 65 hPa, but is not limited thereto.

The nanoparticles present in the diluted solution may maintain an average particle size of 100 to 200 nm, preferably 110 to 190 nm, from before performing the drying until completing the drying.

In one specific embodiment of the present disclosure, the nanoparticles present in the diluted solution maintain an average particle size of 60 to 200 nm from before performing the drying until completing the drying.

In one specific embodiment of the present disclosure, the nanoparticles present in the diluted solution maintain an average particle size of 70 to 190 nm from before performing the drying until completing the drying.

In one embodiment of the present disclosure, the sirolimus has a binding degree with albumin of 80% or more. The binding degree between the sirolimus and albumin can be measured by a person skilled in the art by performing techniques for measuring a binding degree, such as chromatography, centrifugation, and surface plasmon resonance.

In one embodiment of the present disclosure, the albumin is selected from the group consisting of human serum albumin (HSA), bovine serum albumin (BSA), ovalbumin (OVA), recombinant human serum albumin (rHSA), and combinations thereof.

In one embodiment of the present disclosure, the step (c) of mixing and stirring the first solution and the second solution comprises a high-pressure homogenization process.

In one specific embodiment of the present disclosure, the high-pressure homogenization is performed under a pressure condition of 5,000 to 30,000 psi.

In one specific embodiment of the present disclosure, the high-pressure homogenization is performed under a condition of 10,000 to 30,000 psi, 12,000 to 30,000 psi, 15,000 to 30,000 psi, 16,000 to 30,000 psi, 17,000 to 30,000 psi, 18,000 to 30,000 psi, 10,000 to 25,000 psi, 12,000 to 25,000 psi, 15,000 to 25,000 psi, 16,000 to 25,000 psi, 17,000 to 25,000 psi, 18,000 to 25,000 psi, 10,000 to 20,000 psi, 12,000 to 20,000 psi, 15,000 to 20,000 psi, 16,000 to 20,000 psi, 17,000 to 20,000 psi, or 18,000 to 20,000 psi, but is not limited thereto.

The high-pressure homogenization is performed 1 or more times, more specifically 1 to 5 times, 1 to 4 times, or 1 to 3 times, but is not limited thereto.

In one embodiment of the present disclosure, the preparation method further comprises (f) obtaining nanoparticles by filtering and drying the solution having passed through step (e).

In a specific embodiment of the present disclosure, the drying is lyophilization.

In one embodiment of the present disclosure, the lyophilization refers to a process in which nanoparticles, which are a substance to be dried, are frozen, and then the frozen solvent is removed by sublimation in a vacuum environment. The lyophilization process may optionally include an excipient or a lyoprotectant to enhance the storage stability of the lyophilized product.

The excipient or lyoprotectant includes polymers, for example, dextran and polyethylene glycol; saccharides, for example, mannitol, sucrose, glucose, trehalose, and lactose; surfactants, for example, polysorbate; and amino acids, for example, glycine, arginine, and serine, and is preferably mannitol, sucrose, or trehalose, but is not limited thereto.

In one embodiment of the present disclosure, hyaluronidase is added in the lyophilization process.

In the present disclosure, the term 'hyaluronidase' is an enzyme capable of degrading hyaluronic acid, a main component of the extracellular matrix, and although its halflife is very short as 2 minutes, its sustained effect is as long as 24 - 48h, so it increases the administration dose of a drug and helps absorption during subcutaneous injection. The hyaluronidase is derived from mammals, bacteria, leeches, and other parasites. As one of the kinds of such hyaluronidase, there can be mentioned pH20 derived from a mammal, and in the case of intact human-derived pH20, it has a characteristic of being anchored to the cell membrane due to a GPI (glycosylphosphatidylinositol) anchor and is insoluble. Therefore, mammal-derived hyaluronidase is mainly a sheep or bovine testis extract, and is sold as a liquid phase or lyophilized agent, and is manufactured at 1,500 U/ml. Because it is animal-derived, nonhuman protein, etc. may be mixed therein, and an immune response may be caused, so recombinant human hyaluronidase may be used. The recombinant human hyaluronidase is a hyaluronidase of a form improved with a human recombinant gene, and is produced in animal cells such as Genetically engineered Chinese Hamster Ovary (CHO) cells, is soluble, and has versatile utility because it has neutral and acidic pH activities. In addition, compared to animal-derived hyaluronidase, it can be manufactured at a high active concentration of 100,000 U/mg or more.

According to another aspect of the present disclosure, the present disclosure provides nanoparticles comprising sirolimus, albumin, and hyaluronidase.

The nanoparticles according to one aspect of the present disclosure may be prepared by the above-described method for preparing nanoparticles.

In one embodiment of the present disclosure, the sirolimus and albumin are bound to each other.

In one embodiment of the present disclosure, the weight ratio of the sirolimus and albumin is 1:5 to 1:20, but is not limited thereto.

In one embodiment of the present disclosure, the nanoparticles further comprise a lyoprotectant. The lyoprotectant is the same as described above.

In one embodiment of the present disclosure, the nanoparticles may be pharmaceutically formulated in order to be administered to a patient. Therefore, according to still another aspect of the present disclosure, the present disclosure provides a pharmaceutical composition for treating cancer, comprising the nanoparticles.

The pharmaceutical composition may be used for a therapeutic use against a carcinoma for which sirolimus, which is an active ingredient, is used. The carcinoma is, for example, perivascular epithelioid cell tumor (PEComa), but is not limited thereto.

As for the formulation of the pharmaceutical composition, various formulations and drug delivery systems are available in the art. For example, reference may be made to Gennaro, A.R., ed. (1995) Remington's Pharmaceutical Sciences, 18th ed., Mack Publishing Co.

In one embodiment of the present disclosure, the nanoparticles of the present disclosure may be administered as a pharmaceutical composition by one of the following routes: oral; parenteral, for example, transdermal, intranasal, intramuscular, intravenous, subcutaneous, intradermal, intratumoral, etc.

In one embodiment of the present disclosure, the nanoparticles of the present disclosure may include solid, liquid, semi-solid, or gaseous excipients as follows.

The solid pharmaceutical excipients include starch, cellulose, talc, glucose, lactose, sucrose, gelatin, malt, rice, flour, silica gel, magnesium stearate, sodium stearate, glycerol monostearate, sodium chloride, dried skim milk, etc.

The liquid and semi-solid excipients may be selected from glycerol, propylene glycol, water, ethanol and petroleum, various oils including oils of animal, vegetable, or synthetic origin, for example, peanut oil, soybean oil, mineral oil, sesame oil, etc. Preferred liquid carriers, in particular, liquid carriers for an injection solution, include water, saline, an aqueous dextrose solution, and glycols. Other suitable pharmaceutical excipients and their formulations are described in Remington's Pharmaceutical Sciences (Mack Publishing Company, 18th ed., 1990) edited by E. W. Martin.

In a specific embodiment of the present disclosure, the nanoparticles may be formulated for subcutaneous injection.

### Advantageous Effects of Invention

The features and advantages of the present disclosure are summarized as follows:
(a) The present disclosure provides a method for preparing nanoparticles comprising sirolimus and albumin. (b) The present disclosure provides nanoparticles comprising sirolimus, albumin, and hyaluronidase. (c) The present disclosure provides a pharmaceutical composition for treating cancer, comprising the nanoparticles.

When the preparation method of the present disclosure is used, nanoparticles comprising sirolimus and albumin having excellent particle size distribution maintenance stability can be prepared, and the prepared nanoparticles can be prepared into a pharmaceutical composition suitable for subcutaneous injection, thereby improving an increase in an administration dose and the convenience of medication administration.

### Brief Description of Drawings

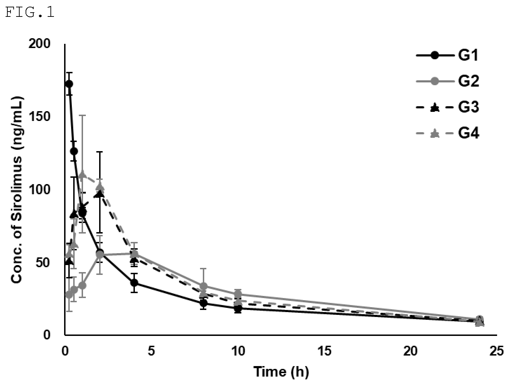

FIG. 1 is a diagram showing the concentration of sirolimus in the blood after intravenous or subcutaneous injection in order to confirm pharmacokinetic information of sirolimus and albumin nanoparticles of the present disclosure.

### DETAILED DESCRIPTION OF THE EMBODIMENTS

Hereinafter, the present disclosure will be described in more detail through examples. These examples are only for illustrating the present disclosure more specifically, and it will be apparent to those of ordinary skill in the art that the scope of the present disclosure is not limited by these examples according to the gist of the present disclosure.

### Examples

Throughout this application, "%" used to indicate the concentration of a specific substance is (weight/weight) % for solid/solid, (weight/volume) % for solid/liquid, and (volume/volume) % for liquid/liquid, unless otherwise specified.

### Example 1: Preparation of nanoparticles comprising sirolimus and albumin

Nanoparticles comprising sirolimus and albumin were prepared as follows.

More specifically, 3.17g of sirolimus was dissolved in chloroform to prepare a sirolimus solution having a concentration of 30% w/v (first solution). In addition, an albumin solution having a concentration of 20% w/v (Greencross Human Serum Albumin Inj. 20%) was diluted to prepare a 12% albumin solution (second solution). The first solution was added to the prepared second solution, and the mixture was mixed in a high-speed homogenizer (IKA, Germany) at 5,000 - 10,000rpm for 5 minutes. The mixture was transferred to a high-pressure homogenizer, and high-pressure homogenized for 2~3 cycle, under a condition of 5,000 - 15,000 psi. The mixture in which high-pressure homogenization was completed was diluted with water in an amount of 6 to 7 times based on volume.

The above-described method was performed in the same manner, but nano particles were prepared while variously changing the concentration of the first solution or the second solution, and the condition of the used multiple amount of water used for dilution, and specific variable conditions for each example are shown in Table 1 below.

**Table 1.**

| Preparation method of nanoparticles comprising sirolimus and albumin by condition | | | |
|---|---|---|---|
| Examples | First solution concentration, w/v % | Second solution concentration, w/v % | Multiples of water used |
| 1-1 | 10.0 | 12.0 | 6 |
| 1-2 | 20.0 | | |
| 1-3 | 30.0 | | |
| 1-4 | 40.0 | | |
| 1-5 | 50.0 | | |
| 1-6 | 60.0 | | |
| 2-1 | 30 | 5.0 | 6 |
| 2-2 | | 10.0 | |
| 2-3 | | 15.0 | |
| 2-4 | | 20.0 | |
| 2-5 | | 30.0 | |
| 3-1 | 30 | 12.0 | 4.0 |
| 3-2 | | | 6.0 |
| 3-3 | | | 8.0 |

### Example 2. Particle size tendency of nanoparticles by process

4.26 g of sirolimus was dissolved in chloroform to prepare a sirolimus solution having a concentration of 44% w/v (first solution). In addition, an albumin solution (Greencross Human Serum Albumin Inj. 20%) having a concentration of 20% w/v was diluted to prepare an albumin solution (second solution,). The first solution was added to the prepared second solution, and the mixture was mixed in a high-speed homogenizer (IKA, Germany) at 5,000 - 10,000rpm for 5 minutes. Subsequently, the mixture was transferred to a high-pressure homogenizer, and high-pressure homogenized for 2~3 cycle, under a condition of 5,000 - 15,000 psi. The mixture in which high-pressure homogenization was completed was diluted with water in an amount of 6 to 7 times based on volume.

After dilution, drying under reduced pressure was progressed using a rotary evaporator, and the organic solvent was removed under a pressure condition in a range of 20 to 50°C and 30 to 60 hPa.

The dispersion from which the organic solvent was removed through drying under reduced pressure was filtered by sterile filtration (0.22um filter), and then filled in a vial to progress lyophilization.

In order to confirm whether particle sizes were formed and stability by process, the particle sizes of the liquid in which the mixture in which high-pressure homogenization was completed was diluted with water, the liquid which underwent drying under reduced pressure, the liquid which underwent sterile filtration, and the liquid rehydrated after lyophilization were measured. The measurement of the particle size was performed using a Zetasizer from Marlvern, and measured using a light scattering method (laser angle was 90 degrees, temperature was 20°C, and solvent was distilled water).

**Table 2.**

| Particle size results of sirolimus and albumin nanoparticles by preparation process | | | |
|---|---|---|---|
| Process | Z-average (nm) | PDI | Peak 1 (volume) |
| The liquid in which the mixture in which high-pressure homogenization was completed was diluted with water | 151.7 | 0.140 | 143.5 |
| The liquid which underwent drying under reduced pressure | 154.9 | 0.132 | 147.7 |
| The liquid which underwent sterile filtration | 146.6 | 0.117 | 135.6 |
| The liquid rehydrated after lyophilization | 191.1 | 0.136 | 193.0 |

As a result of measuring the particle size, it was confirmed that when lyophilization was progressed after sterile filtration, the particle size greatly increased.

Thereafter, in order to rehydrate several lyophilized samples and confirm particle size tendencies and deviations between the samples, particle sizes were measured as shown in Table 3 below.

**Table 3.**

| Particle size results of nanoparticles comprising sirolimus and albumin nanoparticles after lyophilization | | | |
|---|---|---|---|
| No. | Z-average (nm) | PDI | Peak 1 (volume) |
| 1 | 172.0 | 0.096 | 171.9 |
| 2 | 186.5 | 0.126 | 193.1 |
| 3 | 177.8 | 0.127 | 179.0 |
| 4 | 187.2 | 0.119 | 190.8 |
| 5 | 186.9 | 0.129 | 191.2 |
| 6 | 186.3 | 0.083 | 190.8 |
| 7 | 170.1 | 0.110 | 168.1 |
| 8 | 176.3 | 0.123 | 179.9 |

It was confirmed that the particle size of the nanoparticles in the liquid rehydrated after lyophilization increased by about 30 nm to 50 nm, compared to the liquid sterile-filtered before lyophilization. In addition, a deviation was also confirmed between the lyophilized samples progressed at the same time.

### Example 3. Preparation of nanoparticles comprising sirolimus and albumin nanoparticles mixed with a lyoprotectant

A sirolimus solution in which sirolimus was dissolved in chloroform (first solution) an albumin solution (second solution) were mixed in a high-speed homogenizer (IKA, Germany) at 5,000 - 10,000rpm for 5 minutes. Subsequently, the mixture was transferred to a high-pressure homogenizer, and high-pressure homogenized for 2 cycle, under a condition of 5,000 - 15,000 psi. The mixture in which high-pressure homogenization was completed was diluted with water.

After dilution, drying under reduced pressure was progressed using a rotary evaporator, and the organic solvent was removed.

The dispersion from which the organic solvent was removed through drying under reduced pressure was sterile-filtered, and then mannitol, sucrose, and trehalose, which are lyoprotectants, were dissolved to a concentration of 2 to 10% w/v, and then filled in a glass vial to progress lyophilization.

The particle size of the liquid which underwent sterile filtration was measured, and the particle sizes of the lyophilized samples according to the presence or absence of a lyoprotectant were measured.

**Table 4.**

| Particle size results of lyophilized samples according to the presence or absence of a lyoprotectant | | | |
|---|---|---|---|
| Item | Z-average (nm) | PDI | Peak 1 (volume) |
| The liquid which underwent sterile filtration | 166.9 | 0.125 | 165.5 |
| Absence of lyoprotectant | 191.0 | 0.125 | 201.7 |
| Mannitol 2% | 167.7 | 0.125 | 162.5 |
| Mannitol 5% | 165.2 | 0.148 | 159.9 |
| Mannitol 10% | 166.2 | 0.112 | 162.5 |
| Sucrose 2% | 169.7 | 0.134 | 165.3 |
| Sucrose 5% | 167.5 | 0.122 | 162.6 |
| Sucrose 10% | 167.3 | 0.125 | 161.6 |
| Trehalose 2% | 171.0 | 0.115 | 170.0 |
| Trehalose 5% | 167.0 | 0.101 | 164.3 |
| Trehalose 10% | 168.1 | 0.102 | 165.9 |

As a result of the measurement, it was confirmed that an increasing tendency of the particle size was not seen when the lyoprotectants were mixed, except for the 2% concentrations of sucrose and trehalose.

### Example 4. Preparation of nanoparticles comprising sirolimus and albumin mixed with a lyoprotectant and hyaluronidase

Nanoparticles comprising sirolimus, albumin, and hyaluronidase were prepared as follows.

More specifically, 3.17 g of sirolimus was dissolved in 4.3% of chloroform to prepare a sirolimus solution having a concentration of 30% w/v (first solution). In addition, an albumin solution having a concentration of 20% w/v (Greencross Human Serum Albumin Inj. 20%) was diluted to prepare a 12% albumin solution (second solution). The first solution was added to the prepared second solution, and the mixture was mixed in a high-speed homogenizer (IKA, Germany) at 5,000 - 10,000rpm for 5 minutes. Subsequently, the mixture was transferred to a high-pressure homogenizer, and high-pressure homogenized for 2~3 cycle, under a condition of 5,000 - 15,000 psi. The mixture in which high-pressure homogenization was completed was diluted with water in an amount of 6 to 7 times based on volume.

After dilution, drying under reduced pressure is progressed using a rotary evaporator, and the organic solvent is removed under a pressure condition in a range of 20 to 50°C and 30 to 60 hPa.

The dispersion from which the organic solvent is removed through drying under reduced pressure is sterile-filtered, and then dissolved together with a lyoprotectant (mannitol, sucrose, trehalose) at a concentration of 3 to 10 % w/v, and then filled in a vial. Animal-derived hyaluronidase is administered to the corresponding vial at a concentration such as 2000 U/ml (10,000 U per vial) or 4000 U/ml (20,000 U per vial) to progress lyophilization.

**Table. 5**

| Particle size results of lyophilized samples according to the presence or absence of a lyoprotectant and hyaluronidase | | | |
|---|---|---|---|
| Item | Z-average (nm) | PDI | Peak 1 (volume) |
| Mannitol not added Hyaluoronidase not added | 117.3 | 0.155 | 96.3 |
| Mannitol 5%, Hyaluronidase not added | 107.3 | 0.104 | 94.6 |
| Mannitol 5%, Hyaluronidase 2000U/ml | 131.7 | 0.157 | 115.0 |
| Mannitol 5%, Hyaluronidase 4000U/ml | 110.8 | 0.132 | 95.1 |

As shown above, it could be confirmed that when mannitol, which is a lyoprotectant, was not used, the particle size abruptly increased after lyophilization. In addition, in the case of hyaluronidase, it was confirmed that the particle size after lyophilization was maintained smaller when the concentration was 4000 U/ml, which is higher than 2000 U/ml.

### Example 5. Pharmacokinetic experiment of nanoparticles comprising sirolimus and albumin mixed with a lyoprotectant and hyaluronidase

The inventors of the present disclosure prepared albumin nanoparticles comprising a lyoprotectant (mannitol 5%) and hyaluronidase (0, 2000, 4000 U/ml) by the same method as prepared in Example 4, and performed a pharmacokinetic test according to the administration route thereof as follows.

**Table 6.**

| PK experiment information of sirolimus and albumin nanoparticles of the present disclosure | | | | | |
|---|---|---|---|---|---|
| Group (n) | Drug concentration | | Drug dose (Sirolimus , mg/kg) | Drug administration amount (mL/kg) | Administration route |
| | Sirolimus (mg/mL) | Hyaluronidase (U/mL) | | | |
| G1 (3) | 2 | 0 | 2 | 1 | intravenous injection (IV) |
| G2 (3) | | | | | subcutaneous injection (SC) |
| G3 (3) | | 2,000 | | | |
| G4 (3) | | 4,000 | | | |

In the case of Group 1, intravenous injection was performed on SD rats, while for Groups 2 to 4, subcutaneous injection was performed on SD rats, and blood was collected by time slot to measure the concentration of sirolimus in the whole blood. The results are shown in Table 7 and FIG. 1.

**Table 7.**

| Pharmacokinetic parameters (mean value ± standard deviation) by group of sirolimus and albumin nanoparticles of the present disclosure | | | | |
|---|---|---|---|---|
| Pharmacokinetic parameter | G1 (IV) | G2 (SC) (hyaluronidase not administered) | G3 (SC) | G4 (SC) |
| | | | Hyaluronidase Low dose | Hyaluronidase High dose |
| C₀ (ng/mL) | 235.7 ± 9.2 | NA | NA | NA |
| Cₘₐₓ (ng/mL) | NA | 58.9 ± 9.5 | 110.9 ± 17.7 | 119.4 ± 32.0 |
| Tₘₐₓ (h) | 0.3 ± 0.0 | 2.7 ± 1.2 | 1.2 ± 0.8 | 1.7 ± 0.6 |
| t_{1/2} (h) | 14.0 ± 1.0 | 9.4 ± 0.7 | 11.3 ± 1.1 | 10.2 ± 1.6 |
| AUCₗₐₛₜ (ng·h/mL) | 660.8 ± 84.7 | 703.9 ± 83.0 | 748.9 ± 118.8 | 790.6 ± 52.7 |
| Relative AUC (%) | 100.0 | 106.5 | 113.3 | 119.6 |
| Relative Cₘₐₓ (ng/mL) | NA | 100.0 | 188.4 | 202.9 |

As shown in Table 7 and FIG. 1, the degree of drug exposure according to the difference in the administration route compared to the same dosage increased by 6.5% in G2 (subcutaneous injection) compared to G1 (intravenous injection) in terms of Relative AUC, and the degree of drug exposure according to the added amount of hyaluronidase compared to the same dosage and administration route increased by 6.8% in G3 (comprising hyaluronidase) compared to G2 (not comprising hyaluronidase), and 13.1% in G4 (comprising high-dose hyaluronidase) compared to G2 in terms of Relative AUC. This showed a slightly different tendency compared to the data of Herceptin hylecta. According to 'NDA/BLA Multi-Disciplinary Review and Evaluation (761106)', it is explained that in the case of Herceptin hylecta, Tmax is pulled forward (24-29h vs 67h) compared to a formulation not using hyaluronidase, and there is no significant effect on Cmax or AUC. However, the point that can be clearly confirmed in the examples of the present disclosure was that in the case of the nanoparticles of the present disclosure mixed with Hyaluronidase, Tmax was pulled forward, Cmax and AUC increased, and a graph pattern in which an initial burst type graph was gradually released compared to IV was shown.

Therefore, from the above results, it is judged that when hyaluronidase is used, bioavailability can be relatively further increased compared to the case where it is not used, and adverse drug reactions can be reduced because Cmax is remarkably lower compared to the existing IV administration route. When comprehensively considering these results and the like, it can be usefully used as a subcutaneous injection having high medication convenience and patient compliance.

## Claims

1. A method for preparing nanoparticles comprising sirolimus and albumin, comprising the following steps:
(a) preparing a first solution in which sirolimus is dissolved at 5 to 75 w/v%;
(b) preparing a second solution in which albumin is dissolved at 5 to 30 w/v%;
(c) mixing and stirring the first solution and the second solution having a volume ratio of 1: 5 to 1: 70; and
(d) diluting with an aqueous solvent in an amount of 2 to 10 times based on the volume of the solution having passed through step (c).

2. The method for preparing nanoparticles according to claim 1, further comprising (e) removing an organic solvent by drying the diluted solution under reduced pressure.

3. The method for preparing nanoparticles according to claim 2, further comprising (f) obtaining nanoparticles by filtering and drying the solution having passed through step (e).

4. The method for preparing nanoparticles according to claim 3, wherein the drying is lyophilization, and a lyoprotectant is added in the lyophilization process.

5. The method for preparing nanoparticles according to claim 4, wherein hyaluronidase is added in the lyophilization process.

6. The method for preparing nanoparticles according to claim 1, wherein the albumin is selected from the group consisting of human serum albumin (HSA), bovine serum albumin (BSA), ovalbumin (OVA), recombinant human serum albumin (rHSA), and combinations thereof.

7. The method for preparing nanoparticles according to claim 1, wherein the step (c) of mixing and stirring the first solution and the second solution comprises a high-pressure homogenization process.

8. The method for preparing nanoparticles according to claim 7, wherein the high-pressure homogenization is performed under a pressure condition of 5,000 to 30,000 psi.

9. Nanoparticles comprising sirolimus, albumin, and hyaluronidase.

10. The nanoparticles according to claim 9, wherein the sirolimus and albumin are electrostatically bound to each other.

11. The nanoparticles according to claim 9, wherein a weight ratio of the sirolimus and albumin is 1:5 to 1:20.

12. The nanoparticles according to claim 9, further comprising a lyoprotectant.

13. A pharmaceutical composition for treating cancer, comprising the nanoparticles of any one of claims 9 to 12.

14. The pharmaceutical composition according to claim 13, wherein the pharmaceutical composition is a subcutaneous administration formulation, and is a pharmaceutical composition for treating cancer or for use as an immunosuppressant.
